# EUROPEAN PATENT APPLICATION

(11) **EP 1 386 626 A1**
(43) Date of publication of application: **04.02.2004**
(21) Application number: 02388048.7
(22) Date of filing: 31.07.2002
(51) Int. Cl.: A61M 5/142

(54) **Delivery device for treatment of diabetes mellitus**

(71) Applicant: NOVO NORDISK A/S, 2880 Bagsvaerd (DK)
(72) Inventor: Smedegaard, Joergen K., 2720 Vanloese (DK)

(57) **Abstract**

The present invention relates to a delivery device suitable for treatment of diseases or conditions in which a drug has to be applied through the skin of a patient. Thus, a delivery device (1) is provided for delivering a liquid insulin-containing drug into the body of a patient, comprising a reservoir (40) having, in a situation of use, an outlet, an amount of a liquid insulin-containing drug contained in the reservoir (40) expelling means (33,41,52) for expelling the drug out the reservoir (40) through the outlet, and actuating means (20) for actuating the expelling means (33,41,52). In accordance with the invention, the expelling means (33,41,52) upon actuation is adapted for expelling the drug contained in the reservoir (40) during a period of maximum approximately 8 hours.

## Description

### FIELD OF THE INVENTION

The present invention relates to a delivery device suitable for treatment of diseases or conditions in which a drug has to be applied through the skin of a patient. In a more specific aspect, the invention is concerned with a delivery device and a method for the treatment of type 2 diabetes patients.

### BACKGROND OF THE INVENTION

Diabetes mellitus is the common name for at least 2 different diseases, one characterised by immune system mediated specific pancreatic beta cell destruction (insulin dependent diabetes mellitus (IDDM) or type 1 diabetes), and another characterised by decreased insulin sensitivity (insulin resistance) and a functional defect in beta cell function (non-insulin dependent diabetes mellitus (NIDDM) or type 2 diabetes). Whereas the insulin insensitivity does normally not progress, unless body weight increases, the beta cell function deteriorates over the years, as demonstrated in the UK Prospective Diabetes Study (UKPDS) which was a 20-year trial recruiting 5102 patients. The reason(s) for this remains not clear, but a stress situation of the beta cells may be involved. This deterioration can be detected as an overall increase in blood glucose levels, where the rise in night time blood glucose levels results in increased fasting blood glucose levels having an impact on the overall glycaemic control.

The principal treatment of type 1 diabetes is straight forward substitution of the missing insulin secretion, whereas treatment of type 2 is more complicated.

More specifically, in early stages of type 2 diabetes treatment a number of different types of drugs can be used, e.g. drugs which increase insulin sensitivity ("ciglitazones"), decrease hepatic glucose output (e.g. metformin), or reduce glucose uptake from the gut (alfa glucosidase inhibitors), as well as drugs which stimulate beta cell activity (e.g. sulfonylurea/meglitinides). All these drugs are used regularly, and very often in combination with each other. However, the above-described deterioration is reflected in the fact that beta cell stimulators will eventually fail to stimulate the cell, and the patient has to be treated with insulin, either as mono therapy, or in combination with oral medication in order to improve glucose control. Patients with type 2 diabetes also often have problems with dyslipidemia, hypertension, and obesity, all of which are independent risk factors for developing macro-vascular diseases. This means that patients with type 2 diabetes often receive a plethora of drugs.

Insulin treatment as such represents several problems for the patient as well as for the therapist: (i) Presently, insulin has to be injected in order to maintain its full biological potential. The insulin doses needed to control blood glucose excursions will depend on a number of factors, which will vary from day to day, e.g. size, type, and timing of food intake, stomach and gut function as well as exercise level. (ii) The blood glucose levels are also determined by several counter regulatory hormones, the most prominent being glucagon, cortisone, and epinephrine. (iii) Normal insulin secretion is characterised by pulsatile secretion into the portal vein and thereby directly into the liver, just as the secretion is regulated by both neural and humoral factors, as well as a number of nutrients are causing insulin secretion. The most prominent is glucose, but several amino acids, e.g. arginine, and also FFA will cause insulin secretion. (iv) Insulin secretion in connection with meals begins before the rise in blood glucose and the secretion in normal human beings is balanced to the amount of food intake. The primary function of insulin in connection with meals is to a) decrease hepatic glucose output, and b) increase glucose uptake in insulin sensitive tissues (muscle, adipose tissue). Fasting levels of plasma insulin are normally low at a constant level, and as the dose response curve for the muscle starts at a higher level than the curve for adipose tissue, the effect of insulin on glucose uptake in the muscle is minimal, whereas the antilipolytic effect of insulin is maintained.

To better understand the scope of the present invention, a general outline of the current treatment of diabetes will be given.

For type 1 diabetes in general, the state of the art treatment is based on multiple injections, where protracted (i.e. slow acting) insulins are administered once or twice daily, whereas short acting insulin is administered in connection with meals as a bolus injection. A major shortcoming of this treatment is the relative unreliability of the longer acting insulin preparation. The most commonly used insulin is the NPH which is characterized by a relatively rapid and pronounced start of action, and the plasma curve is clearly bell shaped meaning that the patients most of the time either get to much insulin or to little insulin. Newer insulin formulations, (Lantus, insulin detemir) are addressing this issue. A much more stable basal plasma insulin level is obtained by a relatively continuous subcutaneous insulin infusion using a pump. Moreover, the pump can also be used for administering bolus infusions through the same catheter. The effect on overall metabolic control, determined from HbA_{1c} levels, is promising, and pump treatment in this group is able to produce glucose levels within the normal range. The outcome seems slightly better than data obtained with the multiple injection therapy, but both are capable of maintaining patients with good glycaemic control as indicated by HbA_{1c} value in the normal range. A potential benefit when using a pump is the possibility of increasing the basal rate in the early morning hours, where the patient will need a little more insulin due to peaks of counter regulatory hormones. It will represent, however, a practical challenge, as the effect of the counter regulation is not constant and there will therefore be a risk of overdosing. On the other hand, it may turn out to be very useful in certain patients even if the insulin increase is not 100% optimal.

In type 2 diabetes the patients are normally started on oral treatment which is often maintained for too long a period, i.e. with a resulting long period of poor control due to decreasing beta cell function before the appropriate therapy with insulin treatment is started. There are several reasons for this as will be discussed in greater detail below. When eventually the decision to start insulin treatment in patients suffering from diabetes type 2 is taken, different treatment approaches can be followed, however, in recent studies it has been demonstrated that basal, night time insulin administration together with an orally taken drug (e.g. repaglinide or metformin) is superior to mono therapy with the oral drugs in advanced stages of the disease and the treatment is as efficacious as insulin mono therapy. Normally the patient will take an insulin injection (e.g. using NPH insulin) at bedtime and the oral medication during the day (often referred to as combination therapy). The advantages of this are that the patient does not have to take insulin injections during the day, do not have to balance food intake (e.g. size and time) with prandial insulin doses, and gain less weight as compared to full insulin treatment, seeYki-Järvinen H. et al. Comparison of insulin regimens in patients with non-insulin-dependent diabetes mellitus. N. Eng J. Med 1992; 327: 1426-1433, Puhakainen I, Taskinen M-J, Yki-Järvinen H. Comparison of acute daytime and nocturnal insulinization on diurnal glucose homeostasis in NIDDM. Diabetes Care 1994; 17: 805-809, and Kawamori R et al. Fasting plus prandial insulin supplements improve insulin secretory ability in NIDDM subjects. Diabetes Care, 1989; 12: 680-685. Later on many patients may advantageously be treated with a more complete insulin regimen consisting of twice daily injections of an insulin mixture, however there is a tendency towards also using bolus injections in selected patients, as many type 2 patients have severe problems in controlling the post prandial glucose levels. In any case, the earlier the patient is made familiar with insulin injections as a necessary part of the treatment, the better are the chances that the optimal treatment in any given situation can be used as early in the progress of the disease as possible thereby reducing the incidence of both the micro- and macro-vascular complications associated with long-time poor regulation of a type 2 diabetes.

As appears from the above, it would be beneficial if insulin injections could be used as an early supplement to oral drugs in the treatment of type 2 diabetes. However, experience has shown that this very often does not happen, the reasons being many and diverse and based upon concerns originating from both the medical practitioner, typically the patients general practitioner, and the patient.

From the patient's point of view injections are very cumbersome as the patient has to provide and store insulin in a proper way, to carefully remember to take the injections (one cannot count the remaining number of tablets as it is possible with oral treatment), and ultimately the patient has to inject insulin into the body using a needle. The latter action is perhaps the major patient related obstacle to the early introduction of insulin therapy as many people are afraid of needles, find the action both painful and unpleasant and, last but not least, they feel that needle treatment can only be associated with being "seriously ill", an association which is much weaker when taking oral drugs in the form of tablets which is just a "normal" occurrence like taking a tablet against headache or taking vitamins. Further, many patients have heard of and are afraid of hypoglycemia incidences associated with the use of insulin. Another important reason for patients refusing to start insulin treatment early is the fact that most patients at this point of time in their disease actually do not have any significant symptoms or suffer for any complications.

From the medical practitioner's point of view, patient compliance is a major issue as the "relative" need for insulin (i.e. the patient is not in urgent need for treatment with insulin) has to be held up against the risks associated with home treatment using insulin. More specifically, the medical practitioner will be concerned with the risk of overdosing (e.g. the patient taking too large doses and/or taking too many injections) and thereby the risk for hypoglycemic incidences. As many patients are middle-aged or elderly they are not used to, and perhaps do not understand, the kind of accuracy needed in insulin treatment, this in contrast to most diabetes type 1 patients which have started insulin treatment relatively early in life. Further, the insulin profiles achieved with a bedtime injection are not ideal, either resulting in a bell-shaped curve from NPH insulin, or a very flat curve from newly developed protracted insulin types such as Lantus®, where a therapeutic level is build up slowly and where overdosing is a real issue, due to the very long acting profile.

### DISCLOSURE OF THE INVENTION

Having regard to the above-identified problems, it is an object of the present invention to provide a concept for the early insulin treatment of diabetes which is acceptable to both the medical practitioner and the patient, which is safe in use and which will overcome the existing prejudices against needle based insulin treatment. When in the following the term "insulin" is used, this denotes any insulin containing flowable drug or medicament formulation. Thus, the present invention is based on the realization that a device and method should be provided which is easy to use, which to a high degree minimizes the risks of overdosing, and which breaks the patients' "emotional" barriers against insulin injections.

More specifically, the present invention is based on the concept of insulin treatment performed substantially only during the approximately 8 hours during which a patient is at sleep (i.e. typically the night but could, indeed, be any 8 hours during a 24 hour day).

Thus, according to a **first** aspect of the invention, a delivery device is provided comprising a reservoir with insulin as well as means for allowing the contained insulin to be transferred to the body of the patient (user) during approximately 8 hours or less in a controlled manner. The term "controlled" manner indicates that the insulin is delivered substantially in accordance with a predefined rate or profile. The transfer rate may be substantially constant, increasing or decreasing during the infusion period, or having any other desired profile just as the profile may be settable or programmable by the user or the attendant medical person.

The term "controlled" refers to a normal situation of use under normal circumstances, e.g. if the outlet from the reservoir is blocked for some reason, delivery of the preset amount of insulin may not take place or only in part.

The insulin is preferably infused subcutaneously through a small needle in fluid communication with a reservoir outlet. The needle may be attached directly to the outlet opening or via a flexible catheter tubing. As the insulin is infused slowly over a relatively long period, a very fine needle can be used in contrast to the larger needle needed when the same amount of insulin has to be injected using a conventional syringe over a very short period, e.g. 10 seconds. The term infusion is normally associated with delivery through a needle, however, when in the following the term infusion is used instead of the term delivery this is not intended to restrict the disclosure to any specific means for the delivery of a drug to the body of a patient.

In a preferred embodiment the infusion device is in the form of a "patch" comprising a lower surface provided with adhesive means and adapted to be arranged against and attached to a skin surface of the user, an infusion needle being arranged corresponding to the lower surface. In this way the needle is introduced subcutaneously as the infusion device is placed on the skin. As the needle is "hidden" on the lower surface of the device, the patient can mount the device without having to look as the needle, just as the very fine needle assures that insertion is practically painless. In a further preferred embodiment, the infusion device is provided with the needle in a retracted, preferably hidden, position, such that the needle can be advanced after the device has been mounted on the skin. Advantageously, the needle insertion means are combined with actuation means adapted to initiate infusion of the contained insulin.

This arrangement allows the skin-contacting surface to be placed on the skin (this surface being suitably provided with an adhesive coating or the device being provided with some other means of retaining the skin-contacting surface on the skin), and then a relative movement between the base member and the cartridge causes the penetration of the skin (and optionally, the actuation of the expelling means).

In accordance with an important aspect of the present invention, the reservoir contains only a small amount of insulin corresponding to what is needed during the 8 hours the infusion device is adapted to be used, i.e. corresponding to the amount which may otherwise be injected at bedtime as a single injection.

The basal infusion level for a type 2 patient on early combination therapy is determined based on bodyweight and will be in the range of 5 to 50 units (IU), normally 10-40 IU, per 8 hours, depending on insulin sensitivity. As most of the patients are relatively resistant to insulin, it is not imperative to titrate the dose meticulously, and the doctor can have 3-5 night pumps at his disposal, either with the same infusion rate (ml/hour) and different insulin concentrations, or with the same insulin concentration but with different infusion rates.

Thus, in accordance with a **second** aspect of the present invention, an infusion device is provided comprising a reservoir with insulin as well as means for allowing the contained insulin to be transferred to the body of the patient in a controlled manner, wherein the reservoir contains an amount of insulin in the range from 5 to 50 IU, preferably from 10 to 40 IU, and the infusion device is adapted to transfer the insulin during a period of up to approximately 8 hours. The insulin may be fast acting for which a relatively constant infusion rate over approximately 8 hours may be used, or the insulin may be a slower acting type in which the insulin may be infused over a shorter period of time, just as slower acting insulin to a certain degree will "compensate" for any inaccuracies in the infusion rate provided by the infusion device.

To further "tailor" the insulin release to the body and the performance of the infusion device, a mixture of faster and slower acting insulins may be used.

More specifically, traditionally there are four types of insulin, based on how soon the insulin starts working (onset), when it works the hardest (peak time), and how long it lasts in the body (duration). However, each person responds to insulin in his or her own way so onset, peak time, and duration has to be given as ranges. Rapid-acting insulin (e.g. insulin Aspart) reaches the blood within 15 minutes after injection. It peaks 30 to 90 minutes later and may last as long as 5 hours. Short-acting (regular) insulin usually reaches the blood within 30 minutes after injection. It peaks 2 to 4 hours later and stays in the blood for about 4 to 8 hours. Intermediate-acting (NPH and lente) insulins reach the blood 2 to 6 hours after injection. They peak 4 to 14 hours later and stay in the blood for about 14 to 20 hours. Long-acting (ultralente) insulin takes 6 to 14 hours to start working. It has no peak or a very small peak 10 to 16 hours after injection. It stays in the blood between 20 and 24 hours or longer. Some insulins come mixed together, for example regular and NPH insulins, however, this is solely for the purpose of patients using pen or syringe devices for there injections; when using a pump device the object has traditionally been to "mimic" the pancreas for which reason rapid-acting insulin has been used in pumps.

In accordance with a **third** aspect of the invention a system is provided comprising at least two infusion devices each comprising a reservoir with insulin as well as means for allowing the contained insulin to be transferred to the body of the patient in a controlled manner, the reservoir containing from 5 to 50 IU, preferably from 10 to 40 IU, of insulin and the infusion devices being adapted for transferring the contained insulin during a period of up to approximately 8 hours, wherein each reservoir contains a different amount of insulin, thereby allowing a patient to be provided with the most suitable infusion device among the at least two infusion devices. For example, 4 different devices containing respectively 10, 20, 30 and 40 IU may be provided, however, a different number of devices containing different amounts of insulin may be provided.

In the above it is stated that the infusion device comprises means for allowing the contained insulin to be transferred to the body of the patient. These means may take any desirable form providing the desired function, but presently pump arrangements comprising a conveying arrangement connected to the reservoir (i.e. an outlet to be associated with needle infusion means) and including a pressure generating device for feeding the liquid contained in the reservoir by pressure or suction application from the reservoir to the body are preferred for transferring the insulin contained in the reservoir to the patient. In this respect a number of different pump principles may be utilized, e.g. osmotic pumps as known from for example US patents 4,340,048 and 4,552,561, piston pumps as known from for example US patent 5,858,001, membrane pumps as known from for example US patent 6,280,148, flow restrictor pumps (also know as bleeding hole pumps) as known from for example US patents 2,605,765 and 5,957,895, and gas generating pumps as known from for example US patent 5,527,288, which all in the last decades have been proposed for use in durable (refillable) and/or disposable (prefilled) drug infusion systems, however, until now only insulin infusion systems adapted to be used for considerably longer time than 8 hours have been proposed, thereby failing to provide a solution to the above-identified problems associated with the early treatment of diabetes type 2. The infusion device may be provided as a prefilled, entirely disposable device or as a disposable, prefilled portion comprising the reservoir, in combination with a durable control portion adapted to control the pump means which may be entirely or partly incorporated in the disposable portion. In the latter case the patient simply attaches a new disposable pump portion each evening. The control portion may be programmable, which option may or may not be accessible to the patient.

In the context of the present disclosure the term prefilled is used to characterize a device which is supplied to the end-user in a prefilled condition. Preferably the drug reservoir of the device is adapted for not being refillable, e.g. the reservoir does not contain any user-accessible valves or self-sealing filling arrangements allowing for easy refilling.

Due to the limited amount of insulin infused, missing an infusion or having an incomplete infusion will not lead to a major deterioration in the glycaemic control of these patients, as spontaneous development of keto acidosis is not seen in patients with type 2 diabetes, who has a residual endogenous insulin production which although not sufficient to control blood glucose is sufficient to control ketone body metabolism, this in contrast to patients with type 1 diabetes. Moreover, the small total insulin content together with the relative insensitivity to insulin in type 2 patients makes the risk of developing nighttime hypoglycaemia a theoretical issue only. Thus, the infusion device may be of a relatively simple type dispensing with such features as an alarm and additional safety features, this in contrast to (more sophisticated) pumps comprising a larger amount of insulin. Indeed, in case an infusion device comprising a durable control portion is preferred, many such advanced features may be readily incorporated, e.g. flow sensors, alarms, a display etc.

Referring back to the initially identified problems associated with the early introduction of insulin treatment, the above-proposed infusion devices will overcome one or more of these problems. More specifically, from the patient's point of view an infusion device is provided which in its preferred embodiment appears like a simple patch with a hardly recognizable needle having almost no resemblance to traditional insulin injection devices such as syringes and pens, which is easy to use by simply attaching it to a skin surface before going to bed and removing it in the morning. From the medical practitioner's point of view, the risks of overdosing due to the patients taking too large doses and/or taking too many injections are greatly reduced as the patient are provided with infusion devices comprising the prescribed amount of insulin just as the likelihood of applying two patches will be very low. In the same way the risks of underdosing due to the patients forgetting to take the injections are also reduced as it is very easy to check whether the patch has been attached or not.

Correspondingly, in accordance with a **fourth** aspect of the invention a method is provided for the treatment of a patient suffering from diabetes type 2, comprising the steps of: providing an infusion device adapted to infuse an amount of insulin to a patient, establishing at bedtime a fluid communication between the infusion device and the body of the patient, infusing an amount of maximum 50 IU, preferably 10-40 IU, of insulin during a sleep period of approximately 8 hours, and removing the infusion device from the patient after approximately 8 hours.

The infusion device may be in the form of a traditional programmable infusion pump as used for example in pump treatment of type 1 diabetes patients (this may be suitable in certain circumstances such as a trial phase or during hospitalization), however, to fully benefit from the concept of the present invention the infusion device is advantageously of the above-described type, i.e. a prefilled, disposable infusion device specifically adapted to infuse a contained amount of insulin during a maximum of approximately 8 hours.

In the context of the present invention the term expelling means is used as a general term covering situations in which drug is either forced from the reservoir by applying an external force or drawn or sucked from the reservoir using means arranged distally to the reservoir.

### BRIEF DESCRIPTION OF THE DRAWINGS

In the following the invention will be further described with references to the drawings, wherein
fig. 1 shows a perspective view of an infusion device in an initial state,
fig. 2 shows a perspective view of the infusion device of fig. 1 in an actuated state,
fig. 3 shows a "horizontal" cross-sectional view of the infusion device of fig. 2,
fig. 4 shows a first "vertical" cross-sectional view of the infusion device of fig. 2,
fig. 5 shows a second "vertical" cross-sectional view of the infusion device of fig. 2,
fig. 6 shows in detail a flow restrictor, and
fig. 7 shows in detail an infusion needle.

### DESCRIPTION OF A PREFERRED EMBODIMENT

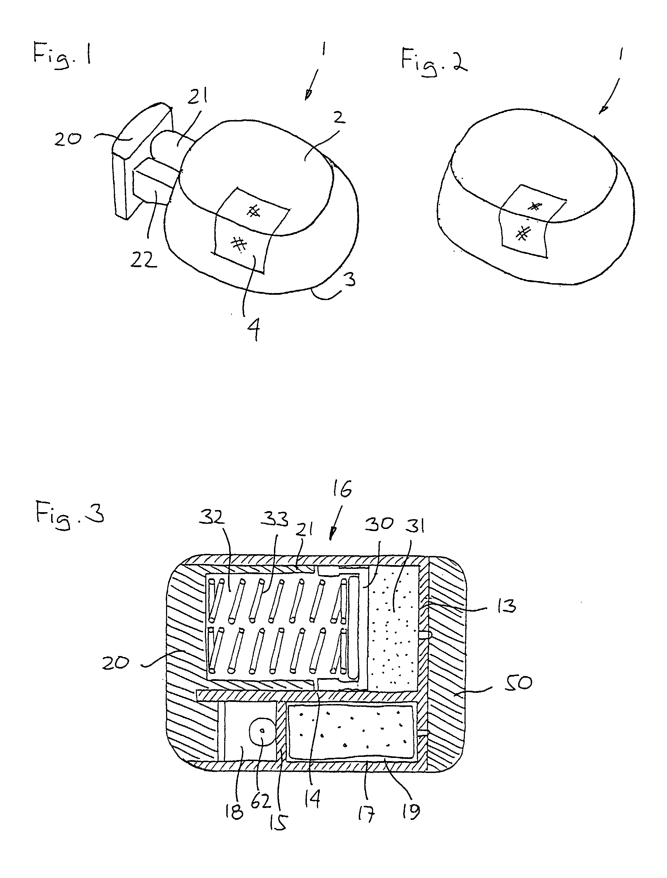

Fig. 1 shows a schematic representation of an embodiment of the invention. Correspondingly, the configuration of the different structures as well as there relative dimensions are intended to serve illustrative purposes only. When in the following terms as "upper", "lower", "right" and "left" or similar relative expressions are used, these only refer to the appended figures and not to an actual situation of use. In the same way the terms "horizontal" and "vertical" refer to planes parallel with respectively perpendicular to a lower surface of the device to be described.

More specifically, fig. 1 shows an infusion device 1 comprising a housing 10 and there from protruding actuation button 20. The housing comprises an upper surface 2 and a lower surface 3 (not to be seen) adapted to be arranged against a skin surface of a user. The upper surface is provided with a transparent window 4 allowing the user to view a drug reservoir arranged within the housing. In fig. 2 the infusion device has been arranged against the skin of a user and the actuation button has pressed into the housing by the user thereby actuating the infusion device as will be explained in detail below.

With reference to fig. 3 the general construction of the infusion device will be described. The housing comprises an upper wall 11, a lower planar base plate 12, side wall portions, an end wall 13 with an outer planar surface, and relative to the latter an opposed open end. Internally the housing comprises a first central wall 14 and a second oblique wall 15 in combination defining three compartments, a drive compartment 16, a reservoir compartment 17 and a needle compartment 18. The drive compartment forms a flat cylinder with an open proximal end and a substantially closed distal end. A piston 30 is slidingly arranged in the cylinder dividing the drive compartment in a distal fluid compartment 31 filled with a viscous drive fluid (e.g. silicon oil), and a proximal spring compartment 32. The actuation button 20 comprises a skirt portion 21 slidingly received in the cylinder thereby closing the spring compartment. In the spring compartment are arranged two helical compression springs 33 acting on the piston, however, any compressible material or member providing a spring action or any other means providing or generating a force (e.g. gas generating means or a liquid/gas mixture) acting on the piston may be utilized. The actuation button further comprises a wedge portion 22 to be received in the needle compartment.

As best seen in fig. 5 the reservoir compartment comprises a flexible drug reservoir 40 with an insulin-containing drug formulation. The reservoir is preferably manufactured from a transparent material allowing the user to view and control the drug through the window 4. In the initial state, i.e. before any drug has been expelled from the infusion device, the reservoir has a configuration substantially corresponding to the configuration of the reservoir compartment, thereby forming a neglectable cavity 19 (or dead-space) between the two components. In case an air filled dead space is not acceptable, the space may be filled with a fluid (for illustrative purposes the gap between the reservoir and the reservoir compartment is relatively large). As appears, the dead-space represents a cavity in a substantially fully collapsed state. Inside the drug reservoir is arranged a U-formed membrane element 41 formed from a self-sealing material and comprising upper and lower membrane portions 42, 43. In the end portion 13 is formed an outlet opening 34 from the fluid compartment and an inlet opening 44 to the reservoir compartment.

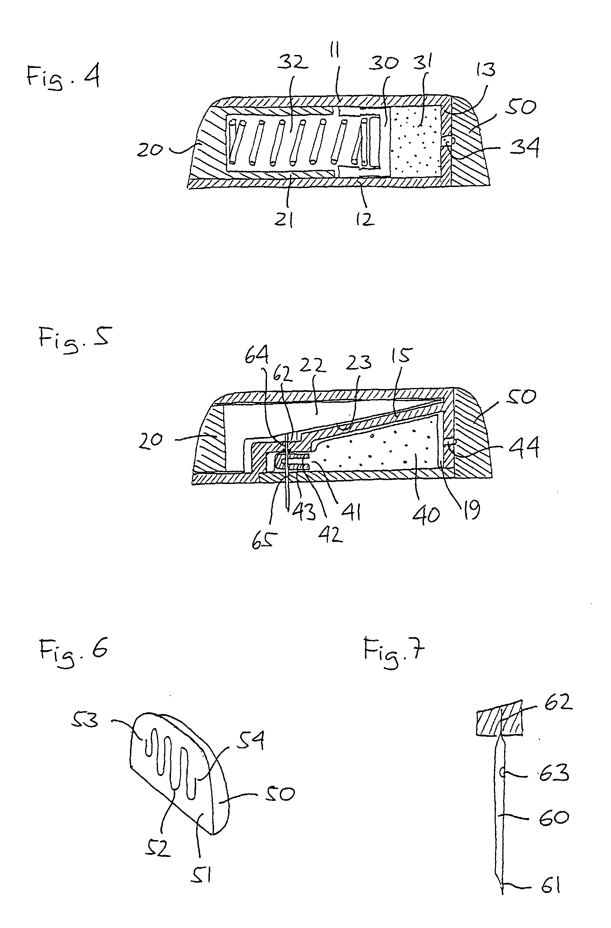

The infusion device further comprises a flow restrictor member 50 (see fig. 6) comprising a planer surface 51 in which a tortuous path 52 is formed between proximal and distal end portions 53, 54. The flow restrictor member 50 is bonded to the outer planar surface of the housing end portion with the proximal and distal end portions in register with the outlet 34 respectively the inlet openings 44. In this way a flow register is formed between the two openings. As appears, the resistance of the flow restrictor, the viscosity of the drive fluid and the force provided by the compressed springs will determine the rate at which the drive fluid will be forced through the flow restrictor to the drug compartment.

The infusion device further comprises a hollow infusion needle 60 as shown in fig. 7, comprising a distal pointed end 61 adapted to be introduced through a skin surface, a closed proximal end at which a needle wedge 62 is formed. In the body of the needle an opening 63 is formed in flow communication with interior of the needle. The proximal end of the needle is arranged in the needle compartment and with the needle body protruding through an opening 64 formed in the first wall 15 into the drug compartment and further into the reservoir. In the initial state (as supplied to the user and not shown in fig. 5) the needle penetrates the upper membrane portion 42 with the distal end 61 arranged between the upper and lower membrane portions 42, 43 inside the reservoir.

Next, with reference to figs. 4 and 5 actuation of the infusion device will be described. When the device has been positioned on a skin surface (preferably the lower surface comprises an adhesive coating) the user actuates the device by fully depressing the actuation button 20 until it locks in place in a recessed position (locking means arranged between the button and the housing is not shown in the figs.) whereby simultaneously the springs 33 are compressed and the wedge portion 22 is moved into the needle compartment. The wedge portion comprises a lower oblique surface 23 in sliding contact with the needle wedge 62 whereby the wedge portion forces the needle downwardly as it is pressed into housing. By this action the pointed distal needle end 61 penetrates the lower membrane portion 43 and is forced out through an opening 65 formed in the base portion. As the infusion device is attached to the skin surface of the user, the infusion needle is hereby introduced through the skin. When the needle is in its fully extended position, the needle opening 63 is positioned between the two membrane portions whereby a fluid communication is established from the drug reservoir to the user. At the same time the drive fluid starts to be expelled from the fluid compartment 31 and through the flow restrictor to the cavity portion 19 where it gradually will compress the flexible reservoir and thereby force out the therein contained insulin-containing drug through the needle and into the user.

Initially air will be expelled from the needle just as air trapped in the flow restrictor and around the drug reservoir (if any) may result in an initial higher infusion rate, however, these effects will be neglectable.

In the shown embodiment the expelling means in form of springs 33 are "energized" during actuation of the device, however, to reduce the force needed to actuate the button 20 the spring means 33 may be pre-tensioned and the drive fluid 31 correspondingly pre-pressurized, whereby alone puncturing of the reservoir by the needle will actuate the expelling means and thereby start infusion.

In the above description of the preferred embodiments, the different structures providing the desired relations between the different components just as the means providing the described functionality for the different components (i.e. force generating means, flow restrictor, flexible reservoir etc.) have been described to a degree to which the concept of the present invention will be apparent to the skilled reader. The detailed construction and specification for the different structures are considered the object of a normal design procedure performed by the skilled person along the lines set out in the present specification.

Although the present disclosure has focused on the treatment of diabetes type 2, the infusion device of the invention may also advantageously be used in the treatment of diabetes type 1. For example, some long-acting (ultralente) insulin types designed to provide an insulin basal rate with a single daily injection will stay in the body for considerable longer than 24 hours. In order to provide the same "functionality" with a type of insulin which will act for a shorter period of time (thereby allowing the doctor to sooner "stop" the treatment) a type of insulin providing a basal rate for approximately 16 hours may be infused over 8 hours, thereby achieving a 24 hour basal rate with all the advantages associated with the infusion device of the present invention, yet providing the doctor with enhanced control of the treatment.

The invention is not restricted to the use of insulin for the treatment of type 2 diabetes, as other drugs may be infused with corresponding advantages in connection with other diseases. For example, growth hormone may advantageously be infused over an 8 hours period during the night in case of growth hormone deficiency.

Although it is preferred that the delivery device of the invention is adapted to infuse the contained insulin over 8 hours, in certain application the infusion may take place during a shorter period, e.g. in case the device is used as an automatic bolus device for infusing a slow acting type of insulin. In other application forms it may be desirable to infused the contained insulin over e.g. approximately 2, 4 or 6 hours.

## Claims

1. A device (1) for delivering a liquid insulin-containing drug into the body of a patient, comprising:
- a reservoir (40) comprising, in a situation of use, an outlet,
- an amount of a liquid insulin-containing drug contained in the reservoir,
- expelling means (33, 41, 52) for expelling the drug out of the reservoir through the outlet,
- actuating means (20) for actuating the expelling means,
wherein the expelling means upon actuation is adapted for expelling the drug contained in the reservoir during a period of maximum approximately 8 hours.

2. A device as defined in claim 1, wherein the drug comprises an amount of insulin in the range of 5-50 IU, preferably 10-40 IU.

3. A device as defined in claim 1 or 2, further comprising a base portion defining a skin-contacting surface for application against the skin of the patient.

4. A device as defined in claim 3, further comprising a delivery needle communicating in a situation of use with the interior of the reservoir and adapted to penetrate the skin of the patient, the delivery needle protruding from the skin-contacting surface.

5. A device as defined in claim 3, further comprising a delivery needle communicating in a situation of use with the interior of the reservoir and adapted to penetrate the skin of the patient, the delivery needle being moveable between a first position in which it is positioned with the device, and a second position in which it is protruding from the skin-contacting surface.

6. A device as defined in claim 5, further comprising needle advancing means for moving the needle from its first to its second position.

7. A device as defined in claim 6, wherein the needle advancing means is associated with the actuating means such that the action of advancing the needle will actuate the expelling means.

8. A device as defined in any of claims 3-7, wherein the base portion comprises adhesive means allowing the device to be arranged against and attached to a skin surface of the patient.

9. A device as defined in any of the previous claims, wherein the reservoir contains a mixture of a faster acting and a slower acting type of insulin.

10. A device as defined in any of the previous claims, comprising first and second portions adapted to releasable engage each other, the first portion comprises the reservoir and the second portion comprising control means, the expelling means being adapted to be controlled by the control means.

11. A system comprising at least two devices, each device as defined in any of the previous claims, wherein each device in its reservoir contains a different amount of insulin, thereby allowing a patient to be provided with the most suitable infusion device among the at least two infusion devices.

12. A method for the treatment of a patient suffering from diabetes type 2, comprising the steps of:
- providing an infusion device adapted to infuse an amount of an insulin-containing drug to a patient,
- establishing at a given time a fluid communication between the infusion device and the body of the patient,
- infusing an amount of maximum 50 IU of insulin during a period of maximum approximately 8 hours, and
- disconnecting the fluid communication between the infusion device and the body of the patient after approximately 8 hours.

13. A method as defined in claim 12, wherein the fluid communication is provided by arranging the infusion device against a skin surface of the patient, and the fluid communication is disconnected by removing the infusion device from the patient.

14. A method as defined in claim 12 or 13, wherein the fluid communication is established at bedtime, the insulin being infused substantially corresponding to a period of sleep.

15. A method as defined in any of claims 12-14, wherein a device (1) as defined in any of claims 1-10 is provided.
